(19) Europäisches Patentamt
European Patent Office
Office européen des brevets

(11) **EP 3 630 023 B1**

(12) **FASCICULE DE BREVET EUROPEEN**

(45) Date de publication et mention
de la délivrance du brevet:
**31.12.2025 Bulletin 2026/01**

(21) Numéro de dépôt: **17730700.6**

(22) Date de dépôt: **31.05.2017**

(51) Classification Internationale des Brevets (IPC):
**A61F 2/60** *(2006.01)*    **A61F 2/74** *(2006.01)*
**A61F 2/64** *(2006.01)*    **A61F 2/50** *(2006.01)*
**A61F 2/68** *(2006.01)*

(52) Classification Coopérative des Brevets (CPC):
**A61F 2/64; A61F 2/68; A61F 2/748;**
A61F 2002/5006; A61F 2002/5033;
A61F 2002/5036; A61F 2002/608; A61F 2002/6854

(86) Numéro de dépôt international:
**PCT/EP2017/063181**

(87) Numéro de publication internationale:
**WO 2018/219450 (06.12.2018 Gazette 2018/49)**

(54) **PROTHÈSE POUR AMPUTÉ FÉMORAL**

PROTHESE FÜR OBERSCHENKELAMPUTIERTE

PROSTHESIS FOR FEMORAL AMPUTEE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(43) Date de publication de la demande:
**08.04.2020 Bulletin 2020/15**

(73) Titulaire: **S & S Sàrl
2740 Moutier (CH)**

(72) Inventeurs:
• **STEINER, Jean-Louis
2740 Moutier (CH)**
• **SCHNEIDER, Clément
2525 Le Landeron (CH)**

(74) Mandataire: **BOVARD AG
Patent- und Markenanwälte
Optingenstrasse 16
3013 Bern (CH)**

(56) Documents cités:
**EP-A1- 2 015 711    EP-A1- 2 730 256
US-A- 6 159 248    US-A1- 2003 125 814**

• **HANS A MAUCH: "Stance Control for Above-
Knee Artificial Legs - Design Considerations in
the S-N-S Knee", BULLETIN OF PROSTHETICS
RESEARCH - FALL 1968, 1 January 1968
(1968-01-01), pages 61 - 72, XP055447030,
Retrieved from the Internet <URL:https://www.
rehab.research.va.gov/jour/68/5/2/61.pdf>
[retrieved on 20180201], DOI: 10.11267/
jspo.27.13**

# Description

## Domaine technique de l'invention

La présente invention se rapporte au domaine des prothèses de genou pour amputé fémoral, et plus particulièrement aux prothèses de genou entièrement mécaniques, c'est-à-dire dépourvues de tout système de capteur et/ou de contrôle électronique.

## État de la technique

Pour permettre de simuler au mieux l'articulation d'un genou sain, des développements récents ont visé à munir les prothèses de systèmes électroniques embarqués sophistiqués pour la détermination et le contrôle de la flexion de la partie tibiale par rapport à la partie fémorale, basées sur des données de position et d'accélération obtenues à l'aide de divers capteurs. Un inconvénient de ce type de solutions est de requérir des processeurs particulièrement efficaces pour traiter les données du capteur et implémenter des algorithmes relativement complexes conçus pour garantir la stabilité du porteur, tout en reproduisant un mouvement le plus naturel possible de la jambe. D'une part, ce genre de solution s'avère particulièrement onéreux pour permettre de garantir systématiquement un temps de réaction suffisamment court du système; par ailleurs, il consomme une énergie non négligeable et requiert un remplacement ou un rechargement fréquent des piles électriques destinées à l'alimenter; enfin, une défaillance électronique pourrait s'avérer catastrophique pour le porteur de la prothèse.

Le document de brevet EP2015711 de la demanderesse consiste en un exemple d'une telle solution pourvue de moyens électroniques, utilisant un capteur placé au niveau du pied et un capteur angulaire au niveau de l'articulation du genou, ainsi qu'un processeur pour traiter les données du capteur.

Le document de brevet US2003/125814 concerne une autre solution de prothèse articulée utilisant un piston dont les propriétés de résistance au niveau du joint entre la partie fémorale et tibiale sont déterminées par les résultats de l'analyse d'un capteur.

Depuis la fin des années soixante, on connaît toutefois par ailleurs d'autres types de prothèses, qui sont a contrario intégralement mécaniques, c'est-à-dire ne requérant l'usage d'aucun capteur ni de système électronique, et ne nécessitent a fortiori aucune alimentation en énergie électrique. Ces prothèses, utilisant essentiellement un piston de préférence hydraulique simulant le groupe musculaire associé notamment aux quadriceps, sont souvent qualifiées de « swing & stance », car elles fonctionnent alternativement selon un mode dit porteur - « stance », où la résistance à la flexion est élevée - et un mode d'oscillation - « swing », où la résistance de l'articulation est réduite et la jambe peut ainsi plus facilement se plier, par exemple, lorsqu'un pas est effectué, suite à la phase de poussée sur une jambe et que celle-ci est amenée vers l'avant. Comme expliqué dans l'article scientifique: Mauch - Stance Control for AK Prothesis, Bulletin of Prothesiscs Research / Fall 1968, hormis un blocage volontaire par l'utilisateur dans le mode porteur de la prothèse pour éviter tout déséquilibre, il existe plusieurs mécanismes de contrôle gérant leur activation alternée de manière automatique dans le cadre d'une démarche normale, et notamment celui basé sur la détection de la pose du talon, dite « heel-control », avec un mode non-porteur par défaut, celui basé sur la flexion du pied et/ou la détection d'une pression sur l'avant du pied, dite « toe-control », avec un mode porteur par défaut, et enfin celui détectant l'application d'une force de compression sur la prothèse, dit « weight control », dont le mode de fonctionnement est relativement proche de celui du « heel-control ». Aucun de ces mécanismes ne s'est toutefois avéré adapté par exemple pour descendre des escaliers, ce qui en rend l'usage peu commode.

Afin de pallier ce problème et améliorer le confort d'usage dans le plus grand nombre de situations possibles correspondant à la vie réelle, y compris la descente d'escaliers, un autre système, dit du type « hyperextension control », a été proposé. Ce mécanisme de contrôle est porteur par défaut lorsqu'un poids est appliqué sur la prothèse, correspondant à la pose du talon au sol, mais réalise l'activation de la phase d'oscillation lorsque la jambe est totalement étendue, c'est-à-dire lorsque la partie tibiale est substantiellement alignée sur la partie fémorale de la prothèse. Autrement dit, un déblocage permettant de plier plus facilement la jambe est censé n'être théoriquement possible qu'à la fin de la phase de poussée sur la jambe, mais jamais à aucun autre moment sinon. Une rééxtension de la jambe est par ailleurs permise, pour effectuer l'oscillation usuelle de la partie tibiale de la jambe, lorsqu'un pas est effectué, à partir d'une position légèrement pliée au moment où le pied n'est plus en appui au sol, grâce à l'action d'une valve qui n'est destinée à se refermer que lorsqu'un poids est appliqué sur la prothèse, et se trouve donc en position ouverte à cet instant.

Un inconvénient de ce type de prothèses décrit ci-dessus est qu'il existe toutefois un risque de déblocage du mode porteur lors d'une extension involontaire de la jambe, et qui risquerait dans ce cas de provoquer sa chute.

Il existe par conséquent un besoin pour une solution exempte de ces limitations connues.

Le document de brevet US6159248 concerne par ailleurs une prothèse pour amputé fémoral contenant une articulation de genou pourvue d'un système de verrouillage permettant de passer d'un mode portant à un mode d'oscillation lorsque le talon est décollé du sol. Aucun système amortisseur n'est toutefois prévu, mais simplement un système de blocage/déblocage permettant d'actionner la phase d'oscillation.

Résumé de l'invention

Un but de la présente invention est de proposer une prothèse dont la fiabilité est améliorée, tout en maintenant des coûts de fabrication et de main d'œuvre réduits.

Selon l'invention, ces buts sont atteints grâce au dispositif de liaison articulée pour prothèse d'amputé fémoral selon la revendication 1, et en particulier les caractéristiques de la partie caractérisante de celle-ci, c'est-à-dire un système de verrouillage intégralement mécanique, agencé pour ne permettre l'activation du deuxième mode de fonctionnement - où la valeur de la résistance correspond à une deuxième valeur minimale, par opposition à la modulaire, aisément adaptable à la plupart des pistons existants prévus pour des prothèses de type « swing and stance » (SNS), sans nécessiter pour autant de modifications structurelles ou d'aménagements additionnels importants sur la prothèse.

Selon un mode de réalisation préférentiel, le dispositif de commutation sécurisé est par ailleurs réglable, en vue de s'adapter au mieux à la démarche du porteur de la prothèse et fiabiliser ainsi au mieux la stabilité lors de l'attaque du talon au sol.

Brève description des dessins

D'autres caractéristiques avantageuses ressortiront plus clairement de la description qui suit d'un mode particulier de réalisation de l'invention donné à titre d'exemple non limitatif et représenté par les dessins annexés, dans lesquels:

- la figure 1 illustre une prothèse connue de l'art antérieur;

- la figure 2 illustre le détail du mécanisme de contrôle de la valve du piston d'une telle prothèse, selon l'art antérieur;

- la figure 3 illustre un schéma de principe montrant les différentes configurations possibles pour la prothèse;

- la figure 4A illustre une vue schématique d'une prothèse selon l'invention, dans une première position de fonctionnement, en mode porteur.

- la figure 4B illustre une vue schématique d'une prothèse selon l'invention, dans une deuxième position de fonctionnement, toujours en mode porteur.

- la figure 4C illustre une vue schématique d'une prothèse selon l'invention, dans une quatrième position de fonctionnement, toujours et encore en mode porteur.

- la figure 4D illustre une vue schématique d'une prothèse selon l'invention, dans une cinquième position de fonctionnement, dans laquelle le mode porteur a été commuté en mode d'oscillation.

- la figure 5 est une vue en coupe d'un pendule modifié pour la commutation entre le mode porteur et le mode d'oscillation de la prothèse selon un premier mode de réalisation préférentiel de l'invention.

- la figure 6 est une vue en coupe d'un pendule modifié pour la commutation entre le mode porteur et le mode d'oscillation de la prothèse selon un deuxième mode de réalisation préférentiel de l'invention.

- la figure 7 est une illustration d'un pendule modifié pour la commutation entre le mode porteur et le mode d'oscillation de la prothèse selon un troisième mode de réalisation préférentiel de l'invention.

Description détaillée

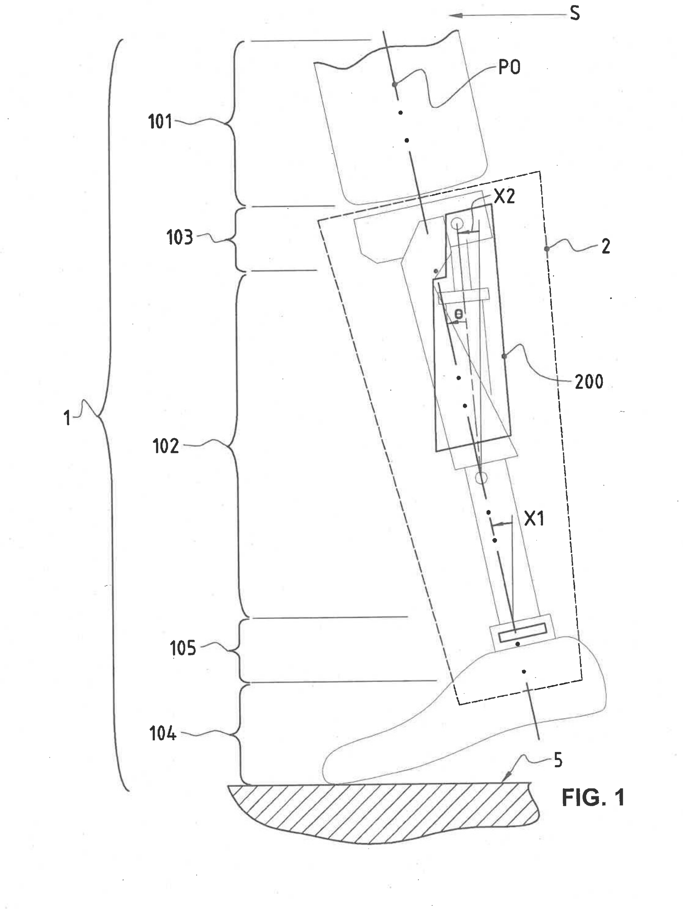

La figure 1 est une vue de profil d'une prothèse de type « Swing and Stance », à laquelle on se référera dans ce qui suit comme simplement une prothèse « SNS ».

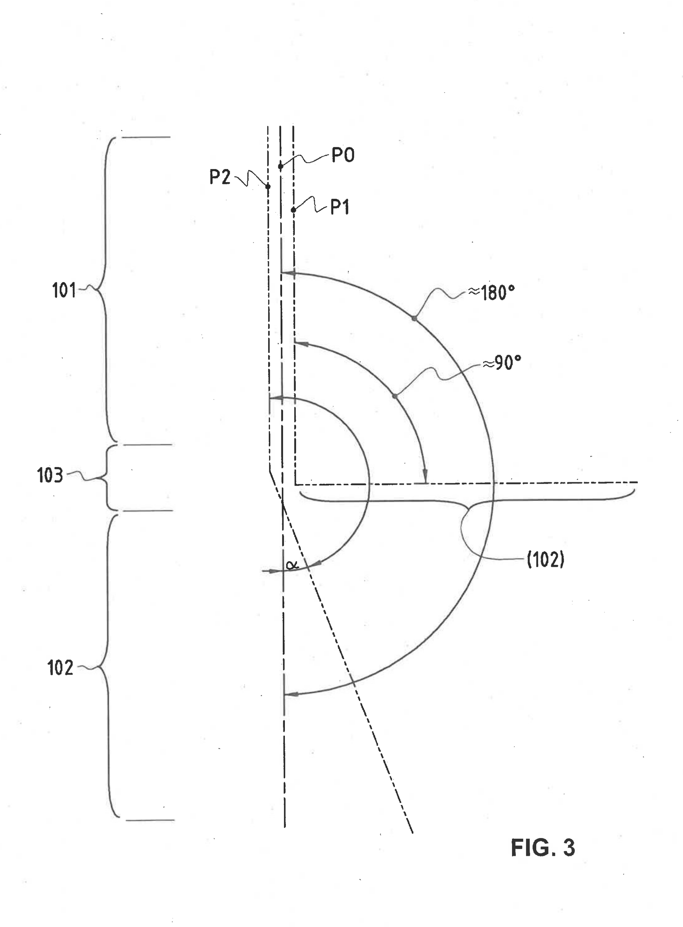

La prothèse 1 comprend classiquement une partie fémorale 101, terminant la cuisse, et destinée à être ajustée sur le moignon de l'amputé, ainsi qu'une partie tibiale 102 remplaçant la jambe de l'amputé, et une partie de pied 104 remplaçant le pied de l'amputé, en vue d'assurer une prise d'appui sur un support 5, typiquement au sol. De manière à assurer une liaison articulée entre la partie fémorale 101 et la partie tibiale 102 de la prothèse, la partie intermédiaire de genou 103 qui les relie comprend un dispositif de liaison articulée 2 simulant cette articulation en fournissant un degré de liberté en rotation limité entre une première position P0 d'hyperextension, dans laquelle la partie fémorale 101 et la partie tibiale 102 s'étendent substantiellement dans le prolongement l'une de l'autre, et une deuxième position P1 de flexion maximale, illustrée plus loin sur la figure 3, dans laquelle la partie fémorale 101 et la partie tibiale 102 forment un angle maximal de flexion prédéfini. Le dispositif de liaison articulée 2 est du reste agencé de manière asymétrique, tout comme un genou sain, et une flexion n'est possible que dans un seul sens de rotation donné.

La prothèse 1 représentée sur la figure 1 comprend également potentiellement une articulation de type cheville 105 entre la partie de pied 104 et la partie tibiale 102; néanmoins le dispositif de liaison articulée 2 selon la présente invention, décrit ci-après, ne nécessite aucunement une telle articulation au niveau du pied et fonctionnerait également avec une prothèse 1 qui en serait dépourvue.

Le dispositif de liaison articulée 2 de la prothèse comprend par ailleurs un mécanisme amortisseur 200, prenant ici la forme d'un piston, destiné à opposer une résistance prédéterminée et variable pour la prothèse 1, en vue de remplacer la cuisse et la jambe de l'amputé de la manière la plus réaliste possible, tout en assurant systématiquement la stabilité au porteur de la prothèse à chaque fois qu'un poids est appliqué sur celle-ci. A cet effet, le piston est susceptible d'être commuté entre un premier mode de fonctionnement M1, sélectionné par défaut, dans lequel la valeur de la résistance est fixée à une première valeur (Vmax) maximale, qui correspond par conséquent au mode dit « porteur » et, un deuxième mode de fonctionnement M2, dans lequel la valeur de résistance est fixée à une deuxième valeur (Vmin) minimale, et qui correspond par conséquent au mode dit « d'oscillation », lequel n'étant censé être actionnable que dans la première position P0 d'hyperextension, dans laquelle est représentée la prothèse sur la figure 1. Sur cette figure, la prothèse 1 est du reste représentée en appui sur la partie avant du pied 104, c'est-à-dire juste avant que ce pied ne quitte le sol lorsqu'un pas est effectué. La prothèse 1 est ainsi légèrement inclinée vers l'avant, et l'angle formé entre la partie tibiale 102 de la prothèse 1 et la verticale forme un premier angle orienté prédéterminé X1 supérieur ou égal à 0 dans le sens de la marche S, qui correspond à la somme du deuxième angle orienté prédéterminé X2 formé entre le piston et la verticale et de l'angle entre le piston et la partie tibiale $\Theta$, habituellement de quelques degrés, au maximum de 5 à 10 degrés,

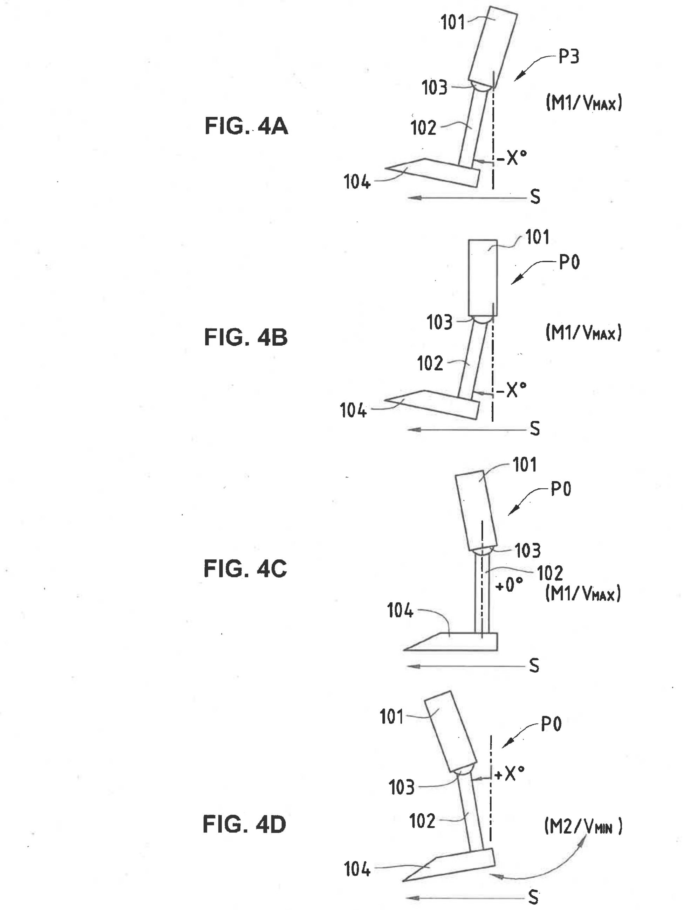

Un objectif majeur de la présente invention est de n'autoriser la commutation du premier mode de fonctionnement M1 au deuxième mode de fonctionnement M2 d'une telle prothèse 1 que dans une situation correspondant à celle illustrée schématiquement par la figure 1, c'est-à-dire dans une situation où la prothèse 1 se trouve certes en hyperextension, mais où la jambe est également légèrement inclinée vers l'avant dans le sens de la marche S, et ce afin d'éviter préemptivement tout déclenchement prématuré du mode d'oscillation, notamment lors de l'attaque du talon au sol ou encore lors d'un déséquilibre du porteur de la prothèse vers l'arrière. La séquence du déroulement d'un pas avec une prothèse 1 munie du dispositif de liaison 2 articulée selon l'invention est décrite par la série de figures 4A,4B,4C,4D.

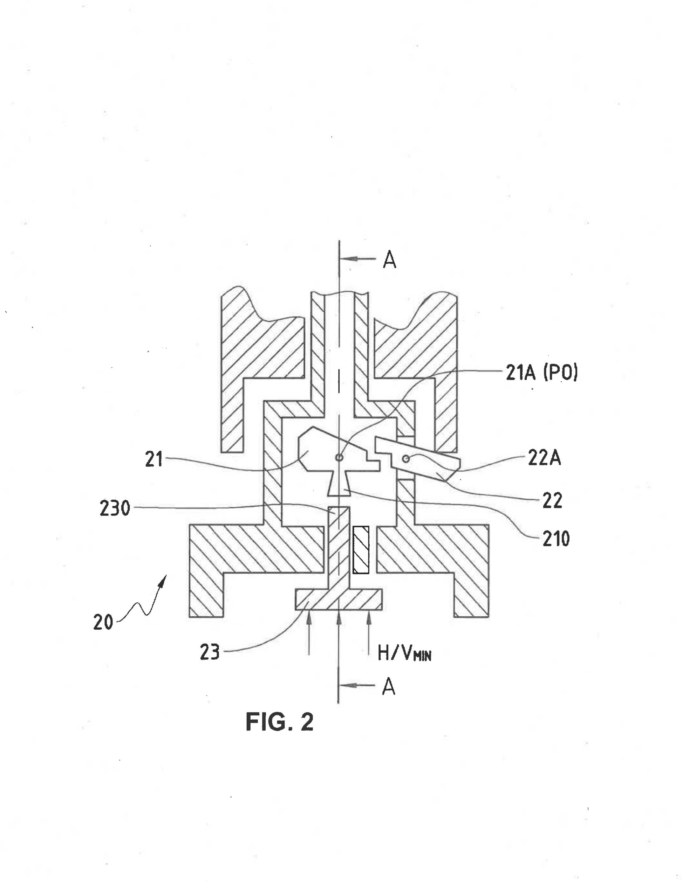

La figure 2 illustre un système de commutation standard 20 mis en œuvre dans le piston d'une prothèse 1 SNS pour le passage du mode porteur au mode d'oscillation, c'est-à-dire du premier mode de fonctionnement M1, choisi par défaut, au deuxième mode de fonctionnement M2. Le piston comprend, monté pivotant sur son axe 21A disposé sur l'axe A-A du piston, un pendule 21 qui fait office de clapet anti-retour pour la valve 23 lors du mode d'oscillation, dans lequel est représenté le piston sur cette figure, grâce à la portion de butée 210 qui coopère sur la tige 230 de la valve 23. Lorsque la prothèse se trouve dans la première position d'hyperextension P0, une force F est exercée par une portion saillante en bordure du piston et fait basculer le contrepoids 22, monté pivotant autour de son axe 22A décalé par rapport à l'axe A-A du piston, ce qui libère ainsi le pendule 21 retenu précédemment et par défaut dans une position verrouillée, permettant la fermeture de la valve 23, garantissant une résistance maximale. La position du pendule 21, suspendu à la verticale mais désormais non bloqué par le contrepoids 22, est telle que la résistance du mécanisme amortisseur 200 formé par le piston est commuté à une valeur minimale Vmin, car malgré la pression hydraulique H exercée sur la valve 23 dans le sens des flèches orientées du bas vers le haut en raison du poids exercé sur la prothèse, cette dernière ne peut plus se refermer et le passage du fluide au niveau des orifices habituellement bloqués par la valve 230 en mode porteur diminue d'autant la résistance exercée par le mécanisme amortisseur 200.

La figure 3 illustre schématiquement les différentes parties d'une prothèse ainsi que les différents angles relatifs entre la partie fémorale 101 et la partie tibiale 102 de la prothèse autour de la partie de genou 103 destinée à remplacer cette articulation. Comme on peut le voir sur cette figure, dans la première position d'hyperextension P0, l'angle entre la partie tibiale 102 et la partie fémorale 103 est plat, c'est-à-dire sensiblement égal à 180 degrés. En pratique, selon les modèles, l'angle entre ces deux parties pourra s'en écarter légèrement, en étant de préférence légèrement supérieur de quelques degrés à cet angle plat en position d'hyperextension de telle sorte que le porteur de la prothèse 1 puisse aisément constater que cette dernière se trouve dans une position charnière permettant le déblocage du mode porteur, c'est la dire permettant la commutation vers le mode d'oscillation.

La figure 3 montre par ailleurs schématiquement la deuxième position P1 pliée de la prothèse, ici selon un angle de flexion maximale d'environ 90 degrés; toutefois, cet angle peut être ajusté en fonction des besoins. On pourra néanmoins remarquer par ailleurs une troisième position intermédiaire P2, qui s'écarte légèrement, de quelques degrés, de la première position d'extension. Le différentiel angulaire $\alpha$ entre ces deux positions, c'est-à-dire la première position d'hyperextension P0 et la troisième position intermédiaire P2, est d'ordinaire de quelques degrés tout au plus, et cette course angulaire correspond à l'avancement additionnel du piston une fois qu'un seuil de résistance due à un ressort, typiquement un ressort de Belleville, a été vaincu, permettant ainsi d'exercer la force F de la figure 2 sur la partie périphérique du contrepoids 22 pour le faire basculer et libérer le pendule 21. Autrement dit, la magnitude de la force F doit dépasser un seuil prédéterminé inhérent à ce ressort, ce qui procure du reste un effet « clic » permettant à l'utilisateur de déterminer si le deuxième mode de fonctionnement M2 d'oscillation est activable ou non.

Dans ce qui suit, on se référera à la série des Figs 4A-4D qui illustrent différentes positions et configurations d'une prothèse 1 modifiée selon un mode de réalisation préférentiel de l'invention lorsqu'un pas est effectué. Bien que ces étapes soient censées se rapprocher le plus possible de ce qui se produit théoriquement avec un genou sain, on pourra toutefois constater que la séquence est légèrement modifiée, puisqu'il est nécessaire de gérer une étape de commutation entre les différents modes de résistance, alors que le groupe musculaire des quadriceps permet de gérer cette résistance de façon continue. Par ailleurs, étant donné que toutes les références relatives aux divers éléments constitutifs de la prothèse 1 illustrés schématiquement sont identiques, elles ne seront pas reprises en détail pour chacune de ces figures.

La figure 4A montre la première étape effectuée lors d'un pas, c'est-à-dire lorsque le talon du pied est posé au sol. Dans cette configuration, la partie tibiale 102 forme un angle orienté de -x° par rapport à la verticale, et seul l'arrière de la partie de pied 104 repose au sol, tandis que la partie fémorale 101 n'est pas encore entièrement alignée sur la partie tibiale 102 au niveau de l'articulation formée par la partie de genou 103. La prothèse 1 se trouve alors typiquement dans la troisième position intermédiaire P3, dans laquelle le deuxième mode de fonctionnement M2 d'oscillation n'est pas encore activable.

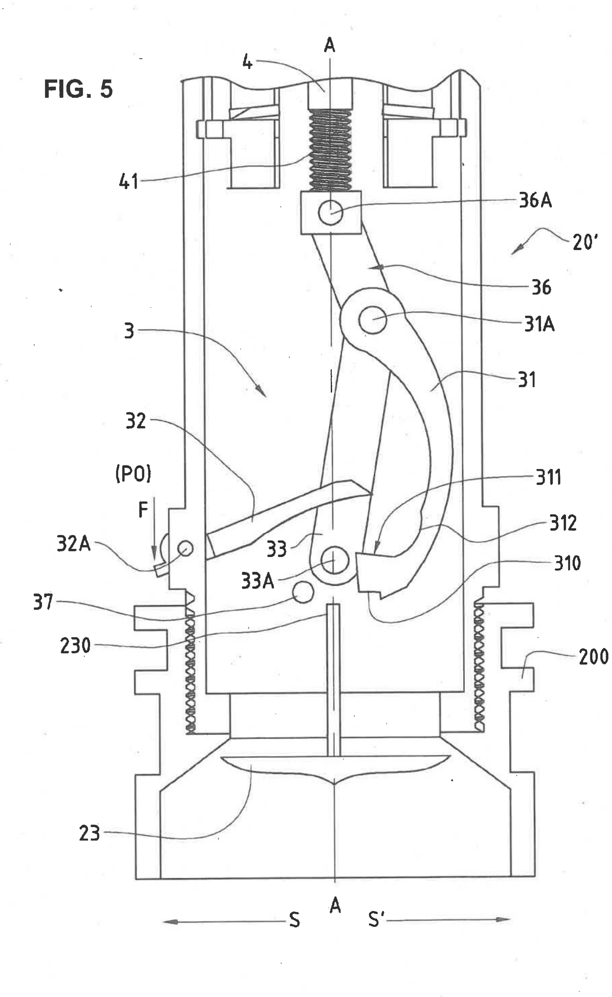

Ensuite, comme illustré sur la figure suivante 4B, en prenant appui sur le talon de la prothèse 1, on peut passer dans la première position d'hyperextension P0 en exerçant une légère pression vers l'arrière du moignon de la cuisse, dans le sens opposé au sens de la marche S, afin que la commutation vers le mode d'oscillation soit possible. L'angle de la partie tibiale 102 par rapport au sol n'a pas changé et forme toujours un angle orienté de -x° par rapport à la verticale. Dans une telle configuration, bien que le deuxième mode de fonctionnement d'oscillation M2 soit théoriquement activable en raison de la position d'hyperextension P0 dans laquelle se trouve la prothèse 1, aucune commutation n'est possible en raison du système de verrouillage 3 décrit plus loin sur les figures 5 à 7.

Ensuite, comme illustré sur la figure suivante 4C, la prothèse 1 se trouve toujours dans la première position d'hyperextension P0, mais elle est basculée, dans son intégralité, vers l'avant dans le sens de la marche S, alors que la partie de pied 104 est intégralement posée au sol, de telle sorte que l'angle entre la partie tibiale 102 et la verticale par rapport au sol est ramené à environ 0 degré. En raison du système de verrouillage 3, la commutation du mode porteur vers le mode d'oscillation est toujours inopérante.

Ce n'est que lors de la dernière séquence illustrée sur la figure 4D que la commutation sera possible. La prothèse 1 se trouve alors toujours et encore dans la première position d'hyperextension P0, mais continue à basculer vers l'avant dans le sens de la marche S, de telle sorte que désormais seule la pointe du pied est en appui au sol et l'angle entre la partie tibiale 102 et la verticale par rapport au sol dépasse un angle positif de +x° degrés par rapport à la verticale. A partir de ce moment-là seulement, la commutation du premier mode porteur, où la résistance du dispositif d'amortissement 200 constitué par le piston est maximale (valeur Vmax), est possible vers le deuxième mode de fonctionnement M2, où la résistance du piston est minimale (valeur Vmin), et la partie tibiale 102 peut osciller vers l'arrière.

Ainsi, le dispositif de verrouillage 3 permet de s'assurer que dans la configuration illustrée par les figures 4B et 4C, constituant des étapes intermédiaires suite à l'attaque du talon au sol, aucun dysfonctionnement ne puisse plus désormais avoir lieu en termes de commutation intempestive de la prothèse d'un mode de fonctionnement vers l'autre, alors que selon les prothèses connues de l'art antérieur, un tel désagrément pouvait se produire, selon la démarche du porteur et le moment où l'hyperextension était déclenchée, puisqu'aucun dispositif de verrouillage ou respectivement de déverrouillage n'était disponible pour fournir de sécurité d'usage additionnelle. Par ailleurs, même lors d'un déséquilibre du porteur de la prothèse 1 vers l'arrière et le déblocage involontaire du contrepoids en passant dans la première position d'hypertextension P0, aucune commutation vers le mode d'oscillation ne sera possible.

Ainsi, selon l'invention, une sécurité additionnelle est fournie par rapport aux prothèses existantes, conditionnant la possibilité de la commutation d'un mode de fonctionnement vers l'autre à la valeur de l'angle que forme la partie tibiable 102 par rapport à la verticale. Dans ce qui suit, on décrira plusieurs variantes, illustrées notamment par les figures 5 à 7, relatives à un mode de réalisation préférentiel pour la réalisation du dispositif de verrouillage 3 selon l'invention, lequel est incorporé dans un piston agissant en tant que mécanisme amortisseur 200, et impliquant un pendule modifié dont le centre de gravité a été décalé par rapport à l'axe de ce piston pour constituer un système de commutation modifié. Une telle variante n'implique qu'un mimimum de modifications structurelles par rapport à une prothèse SNS existante et en facilite par conséquent grandement l'implémentation du système de verrouillage 3, sans nécessiter l'ajout d'un système dédié rajouté remplissant la nouvelle condition de sécurité recherchée. L'ergonomie de la prothèse n'en est par ailleurs pas affectée, aucun aménagement spécial n'étant requis en dehors du piston, ce qui minimise par ailleurs les contraintes en termes de volume requis.

Selon le mode de réalisation préférentiel illustré par les figures 5 à 7, on peut constater que le dispositif de commutation 20 du piston employé en tant que mécanisme amortisseur 200 comprend des éléments fonctionnels similaires à ceux d'un système de commutation 20 standard, à savoir un pendule coopérant alternativement d'une part avec un contrepoids dans le premier mode de fonctionnement (M1), ét d'autre part avec la valve dans le second mode de fonctionnement (M2); néanmoins la modification de la structure de ces éléments leur confère des propriétés physiques différentes altérant les conditions d'activation de la commutation du mode porteur, qui est le mode de fonctionnement par défaut vers le mode d'oscillation, sans toutefois remettre en cause la nécessité de se trouver dans une position d'hyperextension P0 de la prothèse pour rendre possible une telle commutation.

Le même principe physique qui s'applique à tous ces modes de réalisation est de « retarder » l'action du pendule sur la valve lorsqu'un pas est effectué, de telle sorte que sa fonction de clapet anti-retour ne soit activée que lorsque la partie tibiale 102 a déjà basculé dans le sens de la marche S. Ainsi, le centre de gravité du pendule au repos et suspendu depuis la verticale est de préférence décalé, de différentes manières, légèrement vers l'arrière, dans le sens inverse de la marche S', par rapport à l'axe A-A du piston, de telle sorte que sa fonction ne puisse s'activer de préférence que lorsqu'un certain angle d'inclinaison prédéterminé vers l'avant, c'est-à-dire dans le sens de la marche S, soit dépassé pour la partie tibiale 102. En raison du décalage angulaire entre le piston et la partie tibiale 102, cela correspond en pratique à une condition de dépassement d'un autre angle d'inclinaison prédéterminé déterminé par la relation:

$$x > X1 = X2 + \Theta,$$

Où x est l'angle courant de la partie tibiale 102 de la prothèse 1 par rapport au sol, X1 un premier angle orienté prédéterminé dans le sens de la marche et X2 un deuxième angle orienté prédéterminé dans le sens de la marche qui se déduit directement du premier.

Afin de répondre à une réalité physique, en pratique, le premier angle orienté prédéterminé sera de préférence compris entre 0° et 5° degrés, et cet angle sera de préférence réglable afin de pouvoir s'adapter le plus finement possible à la démarche du porteur de la prothèse. Néanmoins, on comprendra que d'autres valeurs angulaires sont possibles sans sortir du cadre de la présente invention.

Dans ce qui suit, étant donné que le corps du piston et de nombreux différents éléments constitutifs du système de verrouillage 3 sont identiques pour les figures 5, 6 et 7, tous les éléments communs à ces figures ne seront pas décrits en détail pour chacune d'entre elles.

La figure 5 décrit ainsi un mode de réalisation préférentiel pour le système de verrouillage 3 intégré dans un système de commutation modifié 20' d'un piston SNS usuel, et qui comprend un pendule modifié 31 de forme sensiblement effilée et arquée, beaucoup plus simple à usiner que le patatoïde usuellement employé pour un tel pendule. Le pendule modifié 31 est monté pivotant autour d'un axe de pivotement 31A décalé vers l'arrière par rapport à l'axe A-A du piston, c'est-à-dire dans le sens inverse de la marche S', de telle sorte que son centre de gravité soit décalé en conséquence et nécessite une inclinaison plus prononcée du piston vers l'avant, c'est-à-dire dans le sens de la marche S, afin qu'il puisse remplir sa fonction de clapet anti-retour pour la valve 23.

Comme sur la figure 2 décrite précédemment, le piston fait toujours office de mécanisme amortisseur 200 qui comprend toujours une valve 23 munie d'une tige 230 à son extrémité supérieure, coopérant avec une portion de butée 310 agencée sur l'extrémité inférieure du pendule modifié 31, tandis qu'une surface d'appui interne 311 est aménagée pour permettre à l'extrémité d'un contrepoids, qui prend ici la forme d'un levier 32 rectiligne pivotant autour de son axe de pivotement 32A agencé sur l'avant du piston, c'est-à-dire dans le sens de la marche S par rapport à l'axe A-A de ce dernier, de venir en appui sur une portion basse du pendule pour verrouiller celui dans une position où le retour de la valve n'est jamais empêché. Le mécanisme de commutation modifié 20' du mécanisme amortisseur 200 étant représenté dans une position d'hyperextension P0 de la prothèse, c'est-à-dire où le changement de mode de fonctionnement est activable en raison de la position levée du levier 32 grâce à l'action de la force F à son autre extrémité. Afin de permettre le mouvement de balancier du levier 32 illustré par les flèches entre sa position de repos et celle de dégagement tel qu'il est représenté sur la figure 5, un dégagement 312 est prévu sur la surface interne du pendule modifié 31, de telle sorte qu'aucun aménagement additionnel n'est nécessaire en dehors du volume du piston.

Comme on peut le voir sur la figure 5, le pendule modifié 31 est par ailleurs agencé à l'extrémité d'un premier bras articulé 33, dont l'axe de pivotement 33A est situé sur l'axe A-A du piston, et dont la position de décalage vers l'arrière dans le sens inverse de la marche S' peut être réglé à l'aide d'un dispositif de réglage 4 comprenant une vis de réglage 41 agencée le long de l'axe A-A et qui est reliée, au niveau de son extrémité inférieure, à un deuxième bras articulé 36 dont l'axe de pivotement 36A est également situé sur l'axe A-A du piston. La longueur relative des différents bras articulés - c'est-à-dire celle du premier bras articulé 33 et celle du deuxième bras articulé 36 - et le pas de la vis de réglage 41 sont configurés de préférence de telle sorte que la commutation envers le deuxième mode de fonctionnement M2, c'est-à-dire le mode d'oscillation de la prothèse 1, ne puisse être déclenché qu'à partir d'une inclinaison de la partie tibiale 102 de la prothèse vers l'avant dans le sens de la marche S de quelques degrés. L'ajustement peut être effectué en continu sur une plage de valeurs d'angle d'inclinaison minimale déterminant le seuil de déclenchement, correspondant au premier angle orienté prédéterminé X1, qui est de préférence strictement positif pour assurer la condition de sécurité additionnelle recherchée, et de préférence compris entre 0° et 5°. Le dispositif de réglage 4 préférentiel illustré gère donc la valeur du premier angle orienté prédéfini (X1) en tant que seuil d'activation pour la commutation vers le mode d'oscillation par l'intermédiaire du décalage de l'axe de pivotement 31A du pendule modifié 31 par rapport à l'axe du piston. Selon le mode de réalisation illustré, la relation entre la valeur de décalage par rapport à l'axe du piston A-A en millimètre et la valeur d'inclinaison additionnelle requise pour permettre l'activation de la commutation n'est toutefois pas linéaire; il a par exemple été constaté qu'un décalage de l'axe du pendule 31A de 2.8 mm conditionnait la valeur du premier angle orienté prédéfini X1 à +2° degrés, tandis qu'un décalage de l'axe du pendule 31A de 3.8 mm conditionnait la valeur du premier angle orienté prédéfini X1 à +5°. On pourra donc partir du principe que le réglage est d'autant plus fin que l'on règle une inclinaison proche de la verticale, soit au début de ce dernier lorsque l'axe de pivotement 31A du pendule est quasiment situé sur l'axe du piston A-A, et la granularité diminuant au fur et à mesure que l'on avance dans le réglage du seuil de déclenchement pour la commutation.

Par ailleurs, afin de maintenir la portion de butée 310 en position de blocage vis-à-vis de la tige 230 de la valve 23 même lorsque le seuil de déclenchement permettant de passer au mode d'oscillation est dépassé, une butée de fin de course 37 est prévue pour limiter l'amplitude du pivotement du pendule modifié 31 vers l'avant, c'est-à-dire dans le sens de la marche S, et s'assurer ainsi qu'il ne puisse jamais reprendre une position où le retour de la valve 23 ne serait plus empêché une fois ce seuil d'inclinaison dépassé; en effet ceci aurait alors pour conséquence de recommuter le dispositif de liaison articulée 2 dans le mode porteur, ce qui n'est évidemment pas souhaitable. Une telle butée de fin de course 37 peut également être utile en cas de mouvement brusque vers l'avant qui génèrerait une forte oscillation du pendule modifié 31 dans le sens de la marche S et pourrait l'amener au-delà de la position de blocage souhaitée empêchant le retour de la valve 23 du piston.

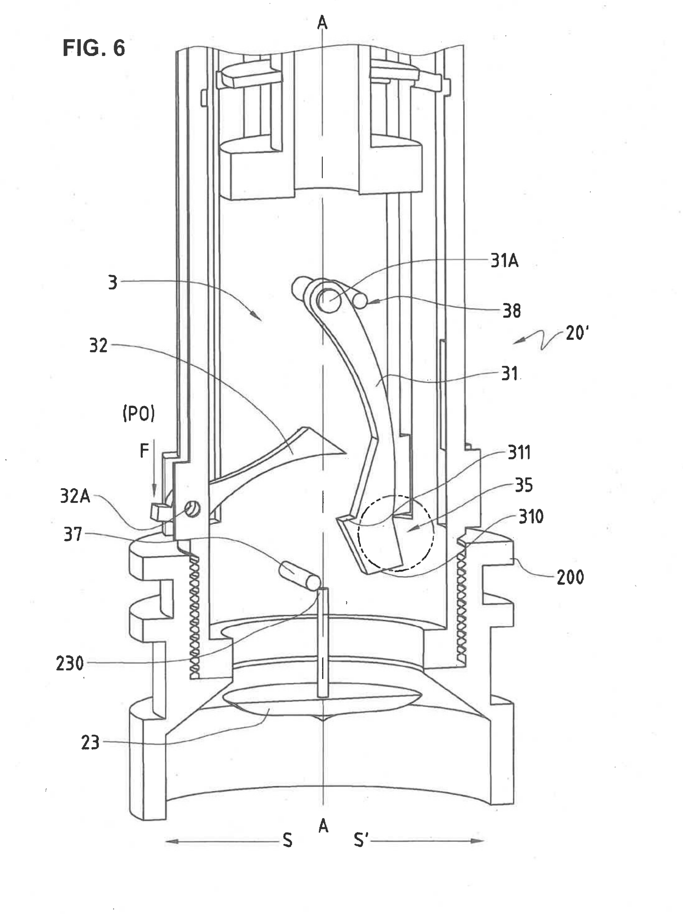

La figure 6 représente un dispositif de commutation modifié 20' selon une variante d'implémentation selon laquelle le pendule modifié 31, au lieu de présenter un axe de pivotement 31A décalé par rapport à l'axe du piston A-A, comme dans la figure 5, afin de décaler le centre de gravité de ce dernier vers l'arrière - c'est-à-dire dans le sens inverse de la marche S' - comprend désormais un balourd, c'est-à-dire une partie lestée 35. L'effet technique obtenu en termes de décalage du centre de gravité du pendule est certes le même, néanmoins, aucun réglage dynamique n'est désormais possible avec une telle variante telle que représentée. La forme géométrique, la disposition de la partie lestée 35 sur le pendule et/ou la modification intrinsèque de la forme du pendule, ainsi que le poids de cette partie lestée pouvant influencer ce décalage, on comprendra qu'une multitude de variantes sont possibles sans sortir du cadre de la présente invention. Afin de fournir un réglage préliminaire, même statique, et une certaine finesse dans la granularité de réglage, on pourra toutefois prévoir des masselottes de différents poids ainsi et prévoir des emplacements d'insertion sur le pendule modifié 31.

Par ailleurs, en raison de l'augmentation de l'inertie du pendule modifié 31, une deuxième butée 38 est prévue, en plus de la butée de fin de course 37, mais cette fois pour limiter l'amplitude d'oscillation du pendule modifié 31 vers l'arrière, c'est-à-dire dans le sens inverse de la marche S'. Cette deuxième butée 38 permet ainsi de compenser ou de minimiser les effets d'inertie du pendule modifié 31 dans le cadre d'une modélisation dynamique, les seuils de déclenchement pour l'activation du changement de mode de fonctionnement, c'est-à-dire le premier angle orienté prédéterminé X1 d'inclinaison de la partie tibiale étant sinon déterminé par des considérations d'équilibrage en statique.

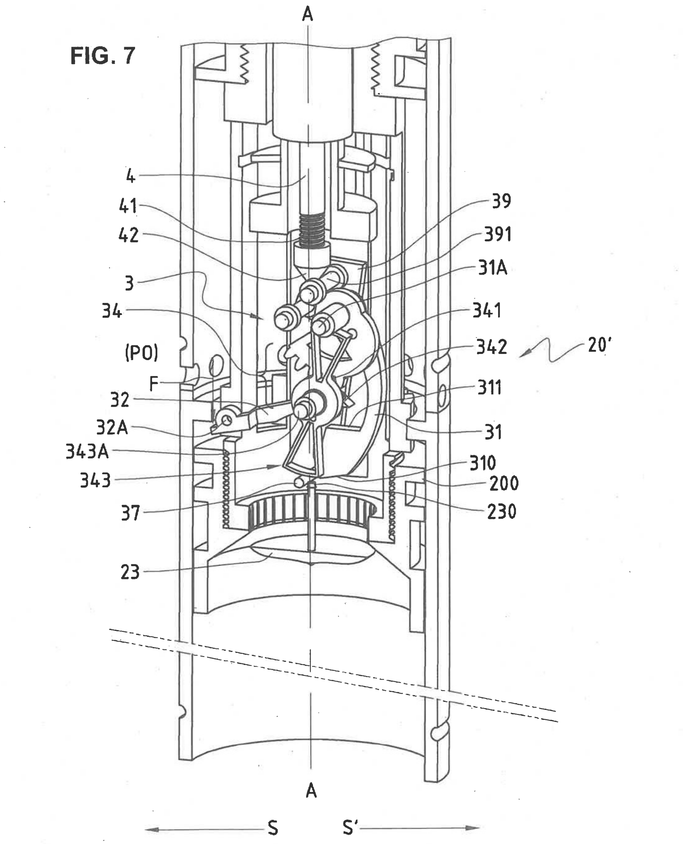

La figure 7 concerne un autre mode de réalisation préférentiel pour la réalisation de l'invention, intégrant toujours le dispositif de verrouillage 3 au système de commutation 20 d'un piston SNS, formant ainsi un système de commutation modifié 20'. Selon cette variante, le pendule modifié 31 est monté sur un support 39 inclinable, dont la position gère le décalage radial de la position de son axe de pivotement 31A par rapport à l'axe du piston 31, similairement à la variante illustrée sur la figure 5 précédemment décrite où un système de bras articulés était utilisé à cet effet. Pour effectuer le réglage, un dispositif de réglage 4 à vis 41, agencé le long de l'axe du piston A-A, est toujours employé, mais qui est désormais muni d'une tête de poussoir 42 effilée venant en appui contre un téton de réglage 391 solidaire du support 39. Ainsi, lorsque la tête de poussoir 42 est déplacée vers le bas, le support 39 est incliné vers l'arrière, c'est-à-dire dans le sens inverse de la marche S', de même que l'axe de pivotement du pendule modifié 31.

Par ailleurs, le système de verrouillage 3 est désormais pourvu d'un mécanisme d'engrenage 34 afin d'obtenir un rapport de démultiplication entre le pivotement d'un mobile 343, monté mobile en rotation sur l'axe de rotation du piston A-A, et celui du pendule modifié 31, afin de ralentir le retour de celui-ci lorsqu'il tend à être amené en position de blocage du retour de la valve. Comme illustré sur la figure 7, le pendule modifié 31 est monté pivotant autour de son axe de pivotement 31A, mais solidaire en rotation d'une pièce munie d'une première portion dentée 341, qui engrène avec une deuxième portion dentée 342, qui elle est solidaire en rotation du mobile 343, dont l'axe de rotation 343A est ici placé directement sur l'axe du piston. L'inertie du mobile 343 est choisie de préférence comme étant largement supérieure à celle du pendule modifié 31, de telle sorte que ce soit le déplacement du mobile 343 qui entraîne celui du pendule, et non l'inverse; par ailleurs, le rapport d'engrenage, par exemple de 2:1 entre le nombre de dents de la deuxième portion dentée 342 solidaire du mobile 343 et celui de la première portion dentée 341 solidaire du pendule modifié 31 est choisi pour conférer un effet levier sur la rotation du pendule modifié 31, en ralentissant d'autant l'amplitude du pivotement de ce dernier selon les proportions du rapport d'engrenage. En réglant les paramètres d'engrenage, on peut ainsi ajuster le premier angle orienté prédéterminé X1 souhaité permettant l'activation du deuxième mode de fonctionnement M2, c'est-à-dire le passage du mode porteur au mode d'oscillation.

On comprendra de la description détaillée qui précède que les modes de réalisation ne sont donnés qu'à titre d'exemple, sans avoir pour vocation d'être exhaustifs pour déterminer le champ de protection pour la présente invention. En particulier, bien que présentée selon une variante préférentielle en tant que module intégrable dans un piston SNS classique, la solution selon l'invention est également compatible avec le développement d'un système intégral, incluant un nouveau piston. Par ailleurs, les caractéristiques avantageuses tirées des modes de réalisations préférentiels décrits pourront être pris isolément ou en combinaison, notamment en ce qui concerne le décalage de l'axe de pivotement 31A du pendule modifié 31, la présence ou non d'une partie lestée 35, ainsi que celle d'un mécanisme d'engrenage 34.

## Revendications

1. Dispositif de liaison articulée (2) pour prothèse (1) d'amputé fémoral, ladite prothèse (1) comprenant au moins:

   - une partie fémorale (101) terminant la cuisse de l'amputé;
   - une partie tibiale (102) remplaçant la jambe de l'amputé;
   - une partie intermédiaire de genou (103) reliant la partie fémorale (101) à la partie tibiale (102);
   - une partie de pied (104) remplaçant le pied de l'amputé, en vue d'assurer une prise d'appui sur un support (5);

   ledit dispositif de liaison articulée (2) étant configuré pour être intégré à ladite partie intermédiaire de genou (103) de manière à assurer une liaison articulée entre ladite partie fémorale (101) et ladite partie tibiale (102) de ladite prothèse (1) entre:

   - une première position (P0) d'hyperextension, dans laquelle ladite partie fémorale (101) et ladite partie tibiale (102) s'étendent substantiellement dans le prolongement l'une de l'autre, et
   - une deuxième position (P1) de flexion maximale, dans laquelle partie fémorale (101) et ladite partie tibiale (102) forment un angle maximal de flexion prédéfini,

   le dispositif de liaison articulée comportant par ailleurs un mécanisme amortisseur (200) destiné à opposer une résistance prédéterminée au moins lors de la flexion de ladite prothèse (1), en remplacement des groupes musculaires sollicités habituellement à cette fin, ledit mécanisme amortisseur (200) étant susceptible d'être commuté entre:

   - un premier mode de fonctionnement (M1), sélectionné par défaut, dans lequel la valeur de la résistance correspond à une première valeur (Vmax) maximale, et
   - un deuxième mode de fonctionnement (M2), actionnable uniquement dans la première position (P0) d'hyperextension, dans lequel la valeur de résistance correspond à une deuxième valeur (Vmin) minimale;

   ledit dispositif de liaison articulée (2) comprend par ailleurs un système de verrouillage (3) intégralement mécanique, incorporé dans un piston agissant en tant que mécanisme amortisseur (200), ledit système de verrouillage (3) étant agencé pour ne permettre l'activation dudit deuxième mode de fonctionnement (M2) qu'à partir d'une certaine inclinaison de ladite partie tibiale (102), correspondant à un premier angle orienté prédéterminé (X1) par rapport à la verticale dans le sens de la marche (S), **caractérisé par** ledit système de verrouillage (3) comprenant un pendule modifié (31) d'un système de commutation modifié (20'), susceptible de coopérer alternativement avec un contrepoids (32) dans le premier mode de fonctionnement (M1), et avec la valve (23) du piston dans le deuxième mode de fonctionnement (M2), ledit pendule modifié comprenant une partie lestée (35) décalant le centre de gravité du pendule dans le sens inverse de la marche (S') par rapport à l'axe du piston (A-A), et/ou étant relié cinématiquement à un système d'engrenage (34), et/ou possédant un axe de pivotement (31A) décalé par rapport à l'axe du piston (A-A).

2. Dispositif de liaison articulée (2) selon la revendication 1, ledit premier angle orienté prédéterminé (X1) étant supérieur ou égal à 0 dans le sens de la marche (S).

3. Dispositif de liaison articulée (2) selon la revendication 1, **caractérisé en ce que** le pendule modifié (31) est configuré de telle sorte que la résistance du dispositif amortisseur (200) est verrouillée dans le premier mode de fonctionnement (M1) tant que l'inclinaison de l'axe du piston ne dépasse pas un deuxième angle orienté prédéterminé (X2) par rapport à la verticale se déduisant directement dudit premier angle orienté prédéterminé (X1) en fonction de l'angle ($\Theta$) formé entre ladite partie tibiale (102) et ledit piston.

4. Dispositif de liaison articulée (2) selon l'une des revendications précédentes, **caractérisé en ce qu'**il contient par ailleurs un dispositif de réglage (4) pour ajuster la valeur dudit premier angle prédéterminé (X1).

5. Dispositif de liaison articulée (2) selon la revendication 4, ledit dispositif de réglage (4) agissant sur le positionnement de l'axe de pivotement (31A) d'un pendule modifié (31).

6. Dispositif de liaison articulée (2) selon la revendication 5, ledit dispositif de réglage (4) comportant une vis (41) actionnant un système de bras articulés dont l'une des extrémités porte ledit axe de pivotement (31A) du pendule modifié (31).

7. Dispositif de liaison articulée (2) selon l'une des revendications 1 à 6, dans lequel le pendule modifié (31) est de forme sensiblement arquée et comprend un dégagement (312) sur sa surface interne afin de permettre le pivotement du contrepoids (32) envers et depuis une surface d'appui (311) lors de la commutation du premier mode de fonctionnement (M1) au deuxième mode de fonctionnement (M2).

8. Dispositif de liaison articulée (2) selon l'une des revendications 1 à 7, **caractérisé en ce qu'**il contient par ailleurs une butée (37) de fin de course pour le pendule modifié (31).

9. Prothèse (1) comportant le dispositif de liaison articulée (2) selon l'une des revendications précédentes.


**Patentansprüche**

1. Gelenkverbindung (2) für eine Prothese (1) für Oberschenkelamputierte, welche Prothese (1) zumindest umfasst:

   - einen Femur-Teil (101), der den Oberschenkel des Amputierten abschließt;
   - einen Tibia-Teil (102), der das Bein des Amputierten ersetzt;
   - einen Knie-Zwischenteil (103), der den Femur-Teil (101) mit dem Tibia-Teil (102) verbindet;
   - einen Fuß-Teil (104), der den Fuß des Amputierten ersetzt, um einen Halt auf einem Untergrund (5) zu gewährleisten;

   wobei die Gelenkverbindung (2) derart konfiguriert ist, in das Knie-Zwischenteil (103) integriert zu werden, um eine gelenkige Verbindung zwischen dem Femur-Teil (101) und dem Tibia-Teil (102) der Prothese (1) zwischen:

   - einer ersten Position (P0) der Überstreckung, in der sich der Femur-Teil (101) und der Tibia-Teil (102) im Wesentlichen in Verlängerung zueinander erstrecken, und
   - einer zweiten Position (P1) der Beugung, in der der Femur-Teil (101) und der Tibia-Teil (102) einen vordefinierten maximalen Beugewinkel bilden,

zu gewährleisten, wobei die Gelenkverbindung außerdem einen Dämpfungsmechanismus (200) umfasst, der dazu bestimmt ist, zumindest bei der Beugung der Prothese (1) einen vorbestimmten Widerstand zu leisten, der die normalerweise zu diesem Zweck beanspruchten Muskelgruppen ersetzt, wobei der Dämpfungsmechanismus (200) zwischen folgenden Modi umgeschaltet werden kann:

   - einen ersten, standardmäßig ausgewählten Betriebsmodus (M1), in dem der Wert des Widerstands einem ersten Maximalwert (Vmax) entspricht, und
   - einem zweiten Betriebsmodus (M2), der nur in der ersten Position (P0) der Überstreckung aktivierbar ist und in dem der Widerstandswert einem zweiten Minimalwert (Vmin) entspricht; und wobei die Gelenkverbindung (2) außerdem ein vollständig mechanisches Verriegelungssystem (3) umfasst, das in einen als Dämpfungsmechanismus (200) fungierenden Kolben integriert ist, wobei das Verriegelungssystem (3) so ausgelegt ist, dass es die Aktivierung des zweiten Betriebsmodus (M2) erst ab einer bestimmten Neigung des Tibia-Teils (102) zulässt, die einem ersten vorbestimmten Winkel (X1) in Bezug auf die Vertikale in Laufrichtung (S) entspricht, **dadurch gekennzeichnet, dass** das Verriegelungssystem (3) ein modifiziertes Pendel (31) eines modifizierten Schaltsystems (20') umfasst, welches abwechselnd mit einem Gegengewicht (32) im ersten Betriebsmodus (M1) und mit dem Ventil (23) des Kolbens im zweiten Betriebsmodus (M2) zusammenwirken kann, wobei das modifizierte Pendel einen beschwerten Teil (35) umfasst, der den Schwerpunkt des Pendels in eine Gegenlaufrichtung (S') in Bezug auf die Kolbenachse (A-A) verschiebt, und/oder kinematisch mit einem Getriebesystem (34) verbunden ist, und/oder eine Schwenkachse (31A) aufweist, die gegenüber der Kolbenachse (A-A) versetzt ist.

2. Gelenkverbindung (2) nach Anspruch 1, wobei der erste vorbestimmte Winkel (X1) in Laufrichtung (S) größer oder gleich 0 ist.

3. Gelenkverbindung (2) nach Anspruch 1, **dadurch gekennzeichnet, dass** das modifizierte Pendel (31) so konfiguriert ist, dass der Widerstand des Dämpfungsmechanismus (200) im ersten Betriebsmodus (M1) gesperrt ist, solange die Neigung der Kolbenachse einen zweiten vorbestimmten Winkel (X2) zur Vertikalen nicht überschreitet, der sich direkt aus dem ersten vorbestimmten Winkel (X1) in Abhängigkeit vom Winkel (Θ) zwischen dem Tibia-Teil (102) und dem Kolben ableiten lässt.

4. Gelenkverbindung (2) gemäß einem der vorstehenden Ansprüche, **dadurch gekennzeichnet, dass** sie außerdem eine Einstellvorrichtung (4) zum Einstellen des Wertes des ersten vorbestimmten Winkels (X1) enthält.

5. Gelenkverbindung (2) gemäß Anspruch 4, wobei die Einstellvorrichtung (4) auf die Positionierung der Schwenkachse (31A) eines modifizierten Pendels (31) einwirkt.

6. Gelenkverbindung (2) gemäß Anspruch 5, wobei die Einstellvorrichtung (4) eine Schraube (41) umfasst, die ein System von Gelenkarmen betätigt, welches an einem Ende die Schwenkachse (31A) des modifizierten Pendels (31) trägt.

7. Gelenkverbindung (2) gemäß einem der Ansprüche 1 bis 6, wobei das modifizierte Pendel (31) im Wesentlichen bogenförmig ist und an seiner Innenfläche eine Aussparung (312) aufweist, um das Schwenken des Gegengewichts (32) zu und von einer Auflagefläche (311) beim Umschalten vom ersten Betriebsmodus (M1) zum zweiten Betriebsmodus (M2) zu ermöglichen.

8. Gelenkverbindung (2) nach einem der Ansprüche 1 bis 7, **dadurch gekennzeichnet, dass** sie außerdem einen Anschlag (37) zur Begrenzung der Bewegung des modifizierten Pendels (31) enthält.

9. Prothese (1) mit der Gelenkverbindung (2) gemäß einem der vorstehenden Ansprüche.


**Claims**

1. 1. Hinged connecting device (2) for a prosthesis (1) for a femoral amputee, the said prosthesis (1) comprising at least:

     - a femoral part (101) ending the thigh of the amputee;
     - a tibial part (102) replacing the leg of the amputee;
     - an intermediate knee part (103) connecting the femoral part (101) to the tibial part (102);
     - a foot part (104) replacing the foot of the amputee, with a view to ensure taking support on a support surface (5);

the said hinged connecting device (2) being configured to be integrated in the said intermediate knee part (103) in such a manner as to ensure an articulated connection between the said femoral part (101) and the said tibial part (102) of the said prosthesis (1) between:

     - a first position (P0) of hyperextension, in which the said femoral part (101) and the said tibial part (102) extend substantially in the extension one from the other, and
     - a second position (P1) of maximal bending, in which femoral part (101) and the said tibial part (102) form a predetermined maximal flexion angle,

the hinged connecting device further comprising a damping mechanism (200) intended to counter a predetermined resistance at least during the bending of the said prosthesis (1), by replacing the muscle groups usually used for this purpose, the said damping mechanism (200) being capable of being switched between:

     - a first operating mode (M1), selected by default, in which the value of the resistance corresponds to a first maximum value (Vmax), and
     - a second operating mode (M2), able to be actuated only in the first position (P0) of hyperextension, in which the resistance value corresponds to a second minimum value (Vmin);

the said hinged connecting device (2) moreover comprises a fully mechanical locking system (3), incorporated into a piston acting as damping mechanism (200), the said locking system (3) being designed to permit the activation of the said second operating mode (M2) only from a certain inclination of the said tibial part (102), corresponding to a first predetermined oriented angle (X1) with respect to the vertical in the direction of walking (S), **characterized by** the said locking system (3) comprising a modified pendulum (31) of a modified switching system (20'), capable of co-operating alternatively with a counterweight (32) in the first operating mode (M1), and with the valve (23) of the piston in the second operating mode (M2), the said modified pendulum (31) comprises a weighted part (35) shifting the center of gravity of the pendulum in the reverse direction of walking (S') with respect to the axis of the piston (A-A), and/or being connected kinematically to a gearing system (34), and/or having a pivot axis (31A) offset with respect to the axis of the piston (A-A).

2. Hinged connecting device (2) according to claim 1, the said first predetermined oriented angle (X1) being greater than or equal to 0 in the direction of walking (S).

3. Hinged connecting device (2) according to claim 1, wherein the said the modified pendulum (31) is configured in such a way that the resistance of the damping device (200) is locked in the first operating mode (M1) as long as the inclination of the axis of the piston does not exceed a second predetermined oriented angle (X2) with respect to the vertical derived directly from the said first predetermined oriented angle (X1) as a function of the angle ($\Theta$) formed between the said tibial part (102) and the said piston.

4. Hinged connecting device (2) according one of the preceding claims, **characterized in that** it further contains an adjustment device (4) for adjusting the value of the said first predetermined angle (X1).

5. Hinged connecting device (2) according to claim 4, the said adjustment device (4) acting upon the positioning of the pivot axis (31A) of a modified pendulum (31).

6. Hinged connecting device (2) according to claim 5, the said adjustment device (4) comprising a screw (41) actuating a system of articulated arms, one end of which bears the said pivot axis (31A) of the modified pendulum (31).

7. Hinged connecting device (2) according to one of the claims 1 to 6, wherein the modified pendulum (31) is of substantially arched shape and comprises a clearance (312) on its inner surface in order to permit the pivoting of the counterweight (32) towards and from a support surface (311) when the first operating mode (M1) is switched to the second operating mode (M2).

8. Hinged connecting device (2) according to one of the claims 1 to 7, **characterized in that** it further contains a stop (37) to limit travel of the modified pendulum (31).

9. Prosthesis (1) comprising the hinged connecting device (2) according to one of the preceding claims.

FIG. 1

**FIG. 2**

FIG. 3

FIG. 4A

FIG. 4B

FIG. 4C

FIG. 4D

FIG. 5

FIG. 6

FIG. 7

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2015711 A
- US 2003125814 A
- US 6159248 A

**Littérature non-brevet citée dans la description**

- *Mauch - Stance Control for AK Prothesis, Bulletin of Prothesiscs Research / Fall*, 1968